# EUROPEAN PATENT SPECIFICATION

(11) **EP 1 368 046 B1**
(45) Date of publication and mention of the grant of the patent: **29.12.2004**
(21) Application number: 02704637.4
(22) Date of filing: 15.03.2002
(51) Int. Cl.: A61K 35/78, A61P 9/00

(54) **PHARMACEUTICAL FORMULATION AND THE USE THEREOF FOR PREPARING A MEDICAMENT FOR THE TREATMENT OF CARDIOVASCULAR DISEASES**
PHARMAZEUTISCHE FORMULIERUNG UND IHRE VERWENDUNG FÜR DIE HERSTELLUNG EINES MEDIKAMENTS ZUR BEHANDLUNG VON HERZ-KREISLAUF-ERKRANKUNGEN
PREPARATION PHARMACEUTIQUE ET UTILISATION DE CETTE DERNIERE POUR PREPARER UN MEDICAMENT SERVANT AU TRAITEMENT DE MALADIES CARDIO-VASCULAIRES

(30) Priority: 15.03.2001 DK 200100444
(43) Date of publication of application: 10.12.2003
(73) Proprietor: M.B. Pharmos A/S, 1022 Copenhagen K (DK)
(72) Inventor: SVENDSEN, Hans, Ole, DK-4180 Soro (DK)
(74) Representative: Klinge, Ulla Callesen
(86) International application number: PCT/DK2002/000177
(87) International publication number: WO 2002/074319

(56) References cited:
- WO-A-98/47376
- SURESH BABU P ET AL: "Hypolipidemic action of curcumin, the active principle of turmeric (curcuma longa) in streptozotocin induced diabetic rats." MOLECULAR AND CELLULAR BIOCHEMISTRY, vol. 166, 1997, pages 169-175, XP002902537
- DATABASE MEDLINE [Online] July 1983 (1983-07) MONSEREENUSORN Y: "Subchronic toxicity studies of capsaicin and capsicum in rats." Database accession no. NLM6622833 XP002902538 & RESEARCH COMMUNICATIONS IN CHEMICAL PATHOLOGY AND PHARMACOLOGY. UNITED STATES JUL 1983, vol. 41, no. 1, July 1983 (1983-07), pages 95-110, ISSN: 0034-5164
- DATABASE BIOSIS [Online] BIOSCIENCES INFORMATION SERVICE, PHILADELPHIA, PA, US; 1991 EL-MASSRY R A ET AL: "STUDIES OF THE EFFECT OF VANILLIN FOOD ADDITIVE ON SOME METABOLIC REACTIONS OF THE EXPERIMENTAL ANIMALS" Database accession no. PREV199294050140 XP002902539 & GRASAS Y ACEITES, vol. 42, no. 6, 1991, pages 428-436, ISSN: 0017-3495
- DATABASE WPI Section Ch, Week 199928 Derwent Publications Ltd., London, GB; Class B04, AN 1999-327818 XP002902540 & CN 1 209 281 A (LI S), 3 March 1999 (1999-03-03)
- DATABASE WPI Section Ch, Week 199219 Derwent Publications Ltd., London, GB; Class A96, AN 1992-147953 XP002902541 & JP 04 091029 A (ZERIA SHINYAKU KOGYO KK) , 24 March 1992 (1992-03-24)

## Description

### Technical Field

The invention relates to a pharmaceutical formulation comprising red pepper, vanillin, gentian root and turmeric as active ingredients. The invention further relates to the use of the formulation for preparing a medicament for the treatment of cardiovascular diseases, and in particular of cholesterol imbalances.

### Background Art

Cardiovascular diseases range among the most serious health problems in the Western world and among the most prevalent causes of death in these countries. These diseases cause those affected thereby loss of quality of life and years and the treatment thereof involves high social costs.

Cardiovascular diseases develop by an interaction between many factors including heredity, life style, dietary and exercise habits. It is known that hypercholesterolemia (elevated cholesterol level in the blood) is associated with an increased risk of cardiovascular diseases. Accordingly much research has been conducted to determine which substances can be used for lowering the cholesterol contents in the blood. It is thus known that exercise and a change of diet to less high-fat foods have a beneficial effect on the level of cholesterol.

Cholesterol is carried through the bloodstream by so-called lipoproteins comprising proteins (apoproteins) and lipids in addition to cholesterol. According to their composition and thus their density, lipoproteins are classified as VLDL (very low-density lipoprotein), LDL (low-density lipoprotein) and HDL (high-density lipoprotein). A high level of LDL cholesterol has proved to increase the risk of cardiovascular diseases, while a high level of HDL cholesterol is said to exert a protective effect.

Even though a change of diet and changed exercise habits to some extent may improve the cholesterol profile, medicaments are still needed, which effectively reduce the level of LDL cholesterol without lowering the HDL cholesterol level and which do not suffer from the adverse effects of the known medicaments.

US 6.022.718 discloses that capsaicinoids, ie capsaicin (8-methyl-N -vanillyl- 6-non-enamide) and dihydrocapsaicin (8-methyl-N-vanillyl-nonanamide) contained in red pepper, have a wide range of physiological effects including a serum cholesterol level-reducing effect.

Indian Journal of Experimental Biology, Sambajah et al., August 1980, vol. 18, pages 898-899, "Hypocholesterolemic Effect of Red Pepper & Capsaicin" describes tests in rats, in which capsaicin and curcumin increased the conversion of cholesterol to bile acids, but also increased the serum cholesterol level. In a corresponding test in rats fed a cholesterol-enriched diet, where red pepper in an amount of 5% was added to the feed, corresponding to 35-40 g for an adult person, a reduction of 22% of the total cholesterol level was obtained.

It appears from Srinivasan M. R., Candrasekhara N. "Comparative Influence of Vanillin and Capsaicin on Liver and Blood Lipids In the Rat." Indian J. Med. Res.; April 1992, B96, 133-35 that vanillin lowered the triglyceride level by 25% and the total cholesterol level by 6%, while the HDL triglyceride level, however, decreased by 11 %.With capsaicin the total cholesterol level was substantially unaffected, the triglyceride level decreased by 13% and the HDL level decreased by 11%.

Ramirez-TortozaM. C. etal. "Oral Administration of a Turmeric Extract Inhibits LDL Oxidation and has Hypocholesterolemic Effects in Rabbits with Experimental Atherosclerosis." Atherosclerosis December 1999; 147(2): 371-8 discloses that turmeric may lower the cholesterol and triglyceride levels using turmeric extract in doses of between 1.66 and 3.2 mg/kg corresponding to 116-224 mg/70 kg body weight. The values of the level decrease are, however, not stated.

The known medicaments have not been able to bring about a significant reduction of the total cholesterol level, the LDL level and the triglyceride level, while maintaining the same or an increased HDL level.

A medicament effectively reducing the LDL cholesterol contents without lowering the HDL cholesterol level is thus still needed.

### Brief Description of the Invention

A surprisingly effective formulation for the treatment of cardiovascular diseases, and in particular of cholesterol imbalances, is obtained by combining red pepper, turmeric, gentian root and vanillin.

The invention thus relates to a pharmaceutical formulation comprising red pepper, vanillin, gentian root and turmeric as active ingredients.

The present invention further relates to the use of the formulation for preparing a medicament for the treatment of cardiovascular diseases.

### Detailed Description of the Invention

In a preferred embodiment the formulation according to the invention comprises a daily intake of

| | |
|---|---|
| 75 - 125 mg of | red pepper |
| 250 - 350 mg of | gentian root |
| 450 - 550 mg of | turmeric |
| 15 - 25 mg of | vanillin |

The daily intake may be taken as a single dose, but may advantageously be taken as several smaller doses, eg two unit doses twice daily.

It has thus surprisingly been found that the combination of active ingredients described above results in a product significantly lowering the LDL cholesterol level without lowering the HDL cholesterol level. In contrast to synthetic hypo-cholesterolemic agents, such as statins, which may suffer from serious adverse effects affecting the liver function, the said product has no essential adverse effects, not even at long-term use thereof.

It is a surprising effect that an intake of such low daily doses of active ingredients brings about such a significant improvement of the cholesterol profile. Furthermore the use of the formulation according to the invention is not encumbered by any of the drawbacks in form of odour problems experienced with for instance garlic-based products. The active ingredients of the formulation are readily accessible, whereby a low-priced product may be obtained.

In addition to the active ingredients the formulation according to the invention comprises one or more conventional auxiliary agents including but not limited to: fillers, lubricants, binders, disintegration agents, colourants and flavouring agents.

The formulation according to the invention may be provided in any suitable conventional administration form including tablets, capsules or powders.

The present invention further relates to the use of the formulation for preparing a medicament for the treatment of cardiovascular diseases. In the present context cardiovascular diseases include ischaemic heart disease and vascular disorders in the brain and the legs. Ischaemic heart disease causes deficient oxygenation of the heart musculature due to a narrowing of the coronary arteries. The narrowing is caused by a thickening of the artery wall due to cholesterol deposits thereon - also named atherosclerosis. The formulation according to the invention has been found to have a beneficial effect on the serum cholesterol level, the LDL cholesterol level being lowered and the HDL cholesterol level remaining unaffected or even increasing. A particular embodiment of the invention thus relates to the use of a formulation according to the invention for preparing a medicament for the treatment of cholesterol imbalances.

The formulation according to the invention may be prepared by a conventional method based on readily accessible starting materials.

Ground red pepper is available in a pharmacological quality from HCI Superfos, Glostrup, Denmark.

Ground turmeric is also available from HCI Superfos, supra.

The other active ingredients, ie gentian root and vanillin, are also readily accessible, eg from HCI Superfos, supra.

The preparation of the formulation according to the invention may be performed by simply blending the ingredients, after granulation thereof if necessary. The composition is then compressed and formulated into the desired administration form.

If tablets or capsules are used, these may optionally be subsequently coated with a pharmacologically acceptable coating, such as a shellac solution.

### Example 1

### The preparation of 1000 tablets.

| **Ingredient** | **Amount, g** |
|---|---|
| ground red pepper | 25 |
| gentian root | 75 |
| turmeric | 125 |
| vanillin | 5 |
| rafteline | 30 |
| icing sugar | 30 |
| Polyvidon K-30 | 4 |
| Ethanol, 96% | q.s.* |
| Purified water | q.s.* |
| potato starch | 64.5 |
| rafteline | 185 |
| microcrystalline cellulose | 62 |
| Instant sugar | 62 |
| silicon dioxide | 7.4 |
| talc | 36.8 |
| magnesium stearate | 7.4 |

| | |
|---|---|
| * = *quantum satis* | |

The active ingredients are granulated, if necessary, and then blended with the remaining ingredients. The mixture is compressed into tablets in a conventional tabletting machine. The tablets are subsequently coated with a shellac solution.

### Pharmacological tests

The effect of the formulation according to the invention was tested in a prospective, double-blind pre-clinical pilot screening of nine participants for a duration of 24 weeks.

The inclusion criteria were as follows:
Persons, whose cholesterol values are within the following ranges:
- total cholesterol > 6 with no other risk factors
- total cholesterol > 5 with one risk factor
- total cholesterol > 4.5 with several risk factors

The risk factors include smoking, overweight/obesity, high blood pressure, diabetes, hereditary predisposition and circulatory disturbances/arteriosclerosis.

The participants were asked not to change their diets, exercise habits or any intake of supplementary nutrients during the test period.

### Chemical analyses

A chemical analysis of the blood lipids was made on the basis of fresh blood at 0, 6, 12 and 24 weeks after an overnight fast The contents in serum of total cholesterol (TS), HDL cholesterol (HDL) and triglyceride (TG) was measured by means of enzymatic standard methods described in Siedel, J. et al., Clin. Chem. 1983,29:1075, Suigiuchi, H. et al., Clin. Chem. 1995, 41:717-723 and Trinder, P.: Ann. Clin. Biochem. 1969, 6:24, respectively.

The serum LDL cholesterol contents was calculated by means of the Friedewald equation, confer Friedewald W.T., Levy R.I., Frederickson D.S.: "Estimation of low density lipoprotein cholesterol concentrations in plasma without the use of preparative ultracentrifuge", Clin. Chem 1992, 18, 499-502:

S-LDL-cholesterol = S-total cholesterol - S-HDL-cholesterol - (S-triglyceride : 5).

The treatment group appears from Table 1 below.

The formulation according to Example 1 was taken in a dosage of two tablets twice daily (morning and night).

### Test results

Tables 2 and 3 show the result of the lipid analyses at the start and after 12 and 24 weeks, respectively. It appears that the formulation according to the invention had a significantly beneficial effect on the total cholesterol contents, viz. a decrease of 12.9% and a decrease of 14.1% of the LDL cholesterol level. The HDL cholesterol level remained unchanged.

The triglyceride level decreased by 13.2%. This value covers wide variations. No participants displayed increased triglyceride levels, but the highest values tended to decrease the most, (50-60%), (data not included).

The effect of the product had no relation to body weight, age, blood pressure, BMI (Body Mass Index), waist/hip measurements and the ratio therebetween (data not shown). This indicates that the participants maintained their dietary habits and energy consumption, for which reason the differences in the lipid values cannot be explained based on changed dietary and exercise habits.

**TABLE 1**

| Baseline characteristics: | |
|---|---|
| | Treatment group |
| Variable | (n=9) |
| Age (years) | 64.4±8.7 |
| | (47-77) |
| Height (m) | 1.74±0.07 |
| Weight (kg) | 77.7±h14.7 |
| BMI (kg/cm²) | 25.63±3.35 (21.1-30.5) |
| Current smokers (number) | 1 |
| Former smokers | 6 |
| Antihypertensive treatment | 1 |
| Energy intake (KJ) | 9902±2403 |
| | (7,470 -13,814) |
| Energy % | |
| - fat | 33.8 |
| - protein | 15.6 |
| carbohydrates | 44.5 |
| - alcohol | 6.1 |

**Table 2**

| The lipid concentrations of the participants treated with the formulation according to Example 1¹) | | | |
|---|---|---|---|
| | **Start** | **Week 12** | **Week 24** |
| Total cholesterol²) | 7.77 ±1.63 | 7.18 ± 1.38 | 6.77 ± 1.28³) |
| HDL cholesterol²) | 1.7 ±0.39 | 1.7 ±0.35 | 1.7 ±0.39 |
| LDL cholesterol²) | 5.77 ±1.54 | 5.2 ±1.38 | 4.8 ± 1.34³⁾ |
| Triglyceride²) | 1.70 ±0.52 | 1.6 ±0.53 | 1.4 ±0.64 |

| | | | |
|---|---|---|---|
| 1) X ± SD, LDL cholesterol calculated by means of the Friedewald equation: LDL=TC -HDL - TG/5 | | | |
| 2) All values are in mmol/l | | | |
| 3) Significant differences from baseline; two paired p = 0.05, Wilcoxon matched pairs test | | | |

**Table 3**

| Changes in percentages in the lipid concentrations at 24 weeks compared to the same concentrations at the start¹⁾ | |
|---|---|
| | Δ |
| Total cholesterol | -12.9²⁾ |
| HDL cholesterol | Unchanged |
| LDL cholesterol | -16. 8²⁾ |
| Triglyceride | -17.6 |

| | |
|---|---|
| 1) All values are X. | |
| 2) Significant differences from baseline; two paired p = 0.05, Wilcoxon matched pairs test | |

## Claims

1. A pharmaceutical formulation comprising red pepper, vanillin, gentian root and turmeric as active ingredients.

2. The formulation according to claim 1 comprising a daily dose of:
| | |
|---|---|
| 75-125 mg of | ground red pepper |
| 15-25 mg of | vanillin |
| 250-350 mg of | ground gentian root |
| 450-550 mg of | ground turmeric |

3. The formulation according to claim 1 or 2, **characterised in that** it further comprises one or more conventional auxiliary agents selected from among fillers, lubricants, binders, disintegration agents, colourants and flavouring agents.

4. A use of the formulation according to any one of the claims 1-3 for preparing a medicament for the treatment of cardiovascular diseases.

5. The use according to claim 4 for preparing a medicament for the treatment of cholesterol imbalances.

## Patentansprüche

1. Pharmazeutische Zubereitung, die roten Pfeffer, Vanillin, Enzianwurzel und Kurkuma als Wirkstoffe umfasst.

2. Zubereitung gemäß Anspruch 1, umfassend eine Tagesdosis von:
| | |
|---|---|
| 75-125 mg | gemahlenem rotem Pfeffer; |
| 15-25 mg | Vanillin |
| 250-550 mg | gemahlener Enzianwurzel; |
| 450-550 mg | gemahlenem Kurkuma. |

3. Zubereitung gemäß Anspruch 1 oder 2, **dadurch gekennzeichnet, dass** sie weiterhin ein oder mehrere herkömmliche Hilfsmittel umfasst, die aus Füllstoffen, Gleitmitteln, Bindemitteln, Sprengmitteln, Farbstoffen und Aromastoffen ausgewählt sind.

4. Verwendung der Zubereitung gemäß einem der Ansprüche 1 bis 3 zur Herstellung eines Medikaments zur Behandlung von Herz-Kreislauf-Erkrankungen.

5. Verwendung gemäß Anspruch 4 zur Herstellung eines Medikaments zur Behandlung von Störungen des Cholesterin-Gleichgewichts.

## Revendications

1. Formulation pharmaceutique comprenant du piment rouge, de la vanilline, de la racine de gentiane et du curcuma en tant que principes actifs.

2. Formulation selon la revendication 1, comprenant une dose journalière de :
75-125 mg de piment rouge moulu
15-25 mg de vanilline
250-350 mg de racine de gentiane moulue
450-550 mg de curcuma moulu

3. Formulation selon la revendication 1 ou 2, **caractérisée en ce qu'**elle comprend de plus un ou plusieurs agents auxiliaires choisis parmi les matières de charge, lubrifiants, liants, agents de désagrégation, colorants et agents aromatisants.

4. Utilisation de la formulation selon l'une quelconque des revendications 1-3 pour la préparation d'un médicament destiné au traitement des maladies cardiovasculaires.

5. Utilisation selon la revendication 4 pour la préparation d'un médicament destiné au traitement des déséquilibres en cholestérol.
